# EUROPEAN PATENT APPLICATION

(11) **EP 3 901 242 A1**
(43) Date of publication of application: **27.10.2021**
(21) Application number: 19899327.1
(22) Date of filing: 05.11.2019
(51) Int. Cl.: C12M 1/32, C12M 1/34, B01L 3/00

(54) **PILLAR STRUCTURE FOR BIOCHIP**

(30) Priority: 17.12.2018 KR 20180162749
(71) Applicant: MBD Co., Ltd., Gyeonggi-do 16229 (KR)
(72) Inventor: KU, Bo Sung, Yongin-si Gyeonggi-do 16939 (KR); LEE, Dong Woo, Daejeon 35381 (KR)
(74) Representative: Goddar, Heinz J.
(86) International application number: PCT/KR2019/014891
(87) International publication number: WO 2020/130335

(57) **Abstract**

The present invention relates to a pillar structure for a biochip. The pillar structure for a biochip according to the present invention is provided to form a biochip for analyzing a sample, which is a biological micromaterial such as a cell, together with a well plate configured to receive a culture solution, and the pillar structure includes: a pillar part having a seating surface on which a cell for culture is placed, and a light irradiation surface configured to be irradiated with light for observing the cell placed on the seating surface; and a plurality of holder parts extending in a state of being spaced apart from the pillar part and having an opening between the adjacent holder parts so that the culture solution is introduced between the holder parts.

## Description

### [Technical Field]

The present invention relates to a pillar structure for a biochip, and more particularly, to a pillar structure for a biochip having an improved structure capable of culturing cells using a relatively large amount of culture solution and thus improving culture reliability and efficiency.

### [Background Art]

A biochip refers to a biological microchip which is also called a bio-device or a biological device and configured such that a sample, which is a biological micromaterial such as DNA, protein, or a cell, is disposed on a substrate so that gene binding, distribution of protein, or a pattern of reaction may be measured and analyzed. The biochips are widely used in fields such as scientific and technological research, new medicine development processes, and clinical diagnosis.

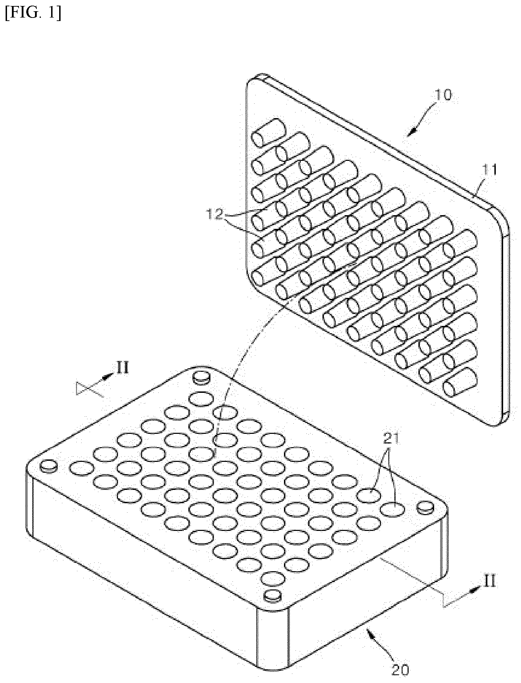

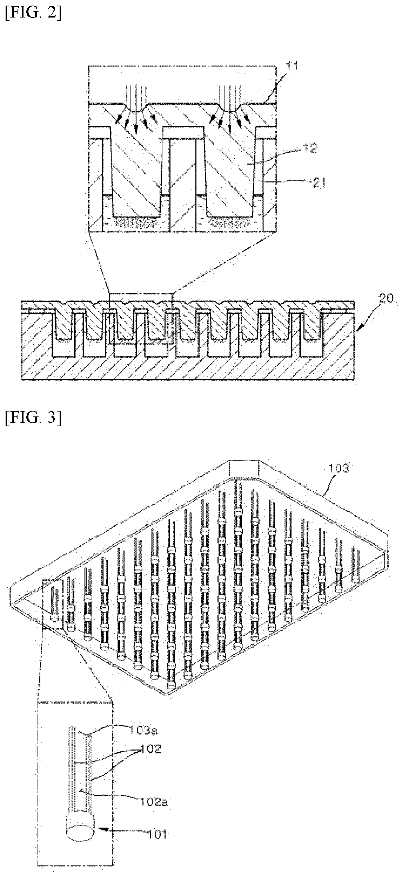

In general, as illustrated in FIGS. 1 and 2, the biochip includes a pillar plate 10 and a well plate 20. FIG. 1 is a perspective view for explaining the biochip in the related art, and FIG. 2 is a cross-sectional view taken along line □-□ in FIG. 1.

As illustrated in FIGS. 1 and 2, the pillar plate 10 of the general biochip has pillar parts 12 integrally provided on one surface of a substrate 11 and each protruding in a column shape. Meanwhile, the well plate 20 includes a plurality of well parts 21 that receives the pillar parts 12, respectively.

A sample is disposed on an end of each of the pillar parts 12 integrally provided on the substrate 11, and a culture solution is received in the well part 21. The general biochip is configured such that as the pillar plate 10 is disposed on the well plate 20, the well part 21 having the culture solution may receive the sample disposed on the pillar part 12. In addition, the biochip is configured such that the sample may be measured by a microscope through the pillar part 12 through which light is transmitted (see FIG. 2).

As the general biochip illustrated in FIGS. 1 and 2 is manufactured by injection molding, the pillar parts 12 are formed integrally with the substrate 11.

In the case of the biochip in the related art, because the pillar part is formed in a cylindrical shape which is a three-dimensional shape, a large amount of culture solution cannot be received in the well part due to a volume of the pillar part, and as a result, there is a problem in that cell culture cannot be smoothly performed.

As a related document, there is Patent No. 10-1816535.

### [Disclosure]

### [Technical Problem]

The present invention has been made in an effort to solve the above-mentioned problem, and an object of the present invention is to provide a pillar structure for a biochip, which is capable of culturing cells with a relatively large amount of culture solution and thus improving culture reliability and efficiency.

### [Technical Solution]

In order to achieve the above-mentioned object, a pillar structure for a biochip according to the present invention is provided to form a biochip for analyzing a sample, which is a biological micromaterial such as a cell, together with a well plate configured to receive a culture solution, and the pillar structure includes: a pillar part having a seating surface on which a cell for culture is placed, and a light irradiation surface configured to be irradiated with light for observing the cell placed on the seating surface; and a plurality of holder parts extending in a state of being spaced apart from the pillar part and having an opening between the adjacent holder parts so that the culture solution is introduced between the holder parts.

The light irradiation surface of the pillar part may be formed between the holder parts, and the seating surface may be provided at a position symmetrical to the light irradiation surface.

The seating surface of the pillar part may be formed between the holder parts, and the light irradiation surface may be provided at a position symmetrical to the seating surface.

The light irradiation surface may be formed to be inclined so that a thickness thereof gradually decreases from a central portion to a portion connected to the holder part.

The holder parts may be formed integrally with a substrate part positioned opposite to the pillar part with the holder parts interposed therebetween, and the substrate part may have a plate-shaped structure.

The holder parts may be manufactured separately from a substrate part having a plate-shaped structure and positioned opposite to the pillar part with the holder parts interposed therebetween, and the pillar structure may further include a connection part serving as a medium for supporting the pillar part and the holder parts on the substrate part, the connection part being formed integrally with the pillar part and holder part and detachably coupled to the substrate part.

### [Advantageous Effects]

According to the pillar structure for a biochip according to the present invention configured as described above, when the cells to be cultured placed on the pillar parts are cultured by the culture solution received in the well plate, the culture solution may be received even in the spaces between the holder parts for connecting the pillar parts to a support body, such that the cells may be cultured with a relatively large amount of culture solution. As a result, there is an advantage in that a rate of culture may be increased, thereby improving culture reliability, and the culture may be performed for a long period of time without an additional supply of the culture solution, thereby improving culture efficiency.

### [Description of Drawings]

FIGS. 1 and 2 are views for explaining a pillar structure for a biochip in the related art.
FIG. 3 is a bottom perspective view illustrating a pillar structure for a biochip according to an exemplary embodiment of the present invention.
FIG. 4 is a cross-sectional view illustrating the exemplary embodiment of the present invention.
FIG. 5 is a cross-sectional view illustrating a pillar structure for a biochip according to another exemplary embodiment of the present invention.
FIG. 6 is an enlarged cross-sectional view illustrating a configuration of a part of a pillar structure for a biochip according to still another exemplary embodiment of the present invention.
FIG. 7 is a bottom perspective view illustrating a pillar structure for a biochip according to yet another exemplary embodiment of the present invention.
FIG. 8 is a view for explaining a through hole formed in a substrate part according to yet another exemplary embodiment of the present invention.

### [Best Mode]

Hereinafter, a pillar structure for a biochip according to an exemplary embodiment of the present invention will be described in detail with reference to the accompanying drawings.

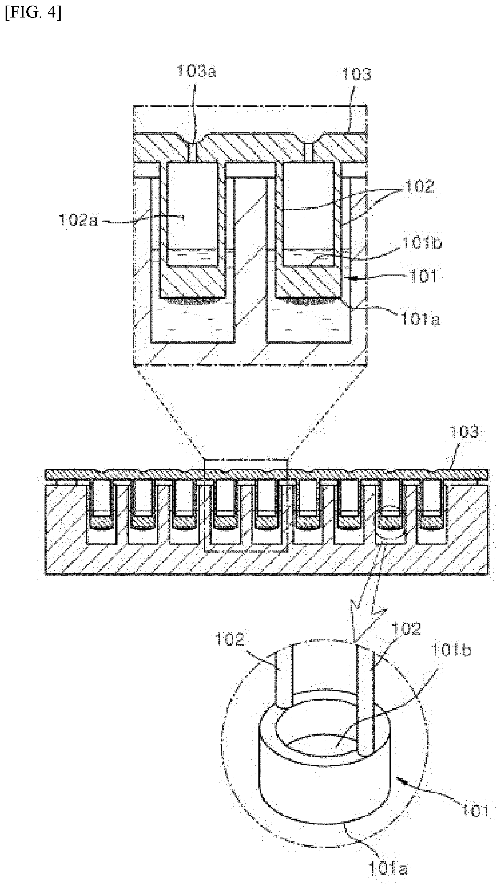

FIG. 3 is a bottom perspective view illustrating a pillar structure for a biochip according to an exemplary embodiment of the present invention, and FIG. 4 is a cross-sectional view illustrating the exemplary embodiment of the present invention.

As illustrated in FIGS. 3 and 4, the pillar structure for a biochip according to the exemplary embodiment of the present invention is provided to form a biochip for analyzing a sample, which is a biological micromaterial such as a cell, together with a well plate configured to receive a culture solution. The pillar structure for a biochip includes pillar parts 101 and a plurality of holder parts 102.

The pillar part 101 includes a seating surface 101a on which a cell to be cultured is placed, and a light irradiation surface 101b provided opposite to the seating surface 101a on the basis of an imaginary division line that divides the pillar part 101 into two portions. The light irradiation surface 101b is a portion irradiated with light for observing the cell placed on the seating surface 101a.

As well illustrated in FIG. 3, the plurality of pillar parts 101 may be prepared in order to culture many cells at a time. Therefore, in general, the plurality of pillar parts 101 is arranged in longitudinal and transverse directions at predetermined intervals on a single substrate part 103. However, the present invention is not limited thereto, and the pillar structure for a biochip according to the present exemplary embodiment may of course be implemented by a configuration in which only the pillar parts 101 and the holder parts 102 connected to the pillar parts 101 are provided without the substrate part 103.

As well illustrated in FIG. 4, the holder parts 102 are spaced apart from each other and extend from the pillar part 101, an opening 102a is formed between the adjacent holder parts 102, such that the culture solution is introduced between the holder parts 102 through the opening 102a.

The holder parts 102 employed in the present exemplary embodiment are provided as a pair at positions facing each other. However, the present invention is not limited thereto, and for example, three holder parts may be provided at an interval of 120 degrees.

Further, the holder parts 102 may be formed integrally with the substrate part 103 which is positioned opposite to the pillar parts 101 with the holder parts 102 interposed therebetween and has a plate-shaped structure.

Meanwhile, the substrate part 103 may include through holes 103a that enable air to be smoothly discharged during injection molding. The through holes 103a enable air to be smoothly discharged during injection molding according to the present exemplary embodiment, thereby enabling precise product molding.

According to the pillar structure for a biochip according to the exemplary embodiment of the present invention configured as described above, when the cells to be cultured placed on the pillar parts 101 are cultured by the culture solution received in the well plate, the culture solution may be received even in the spaces between the holder parts 102 for connecting the pillar parts 101 to a support body, such that the cells may be cultured with a relatively large amount of culture solution. As a result, there is an advantage in that a rate of culture may be increased, thereby improving culture reliability, and the culture may be performed for a long period of time without an additional supply of the culture solution, thereby improving culture efficiency.

The light irradiation surface 101b of the pillar part 101 may be formed between the holder parts 102 in order to prevent the light from interfering with the holder parts 102. The seating surface 101a may be provided at a position symmetrical to the light irradiation surface 101b because the cell placed on the seating surface 101a is observed through the light irradiation surface 101b.

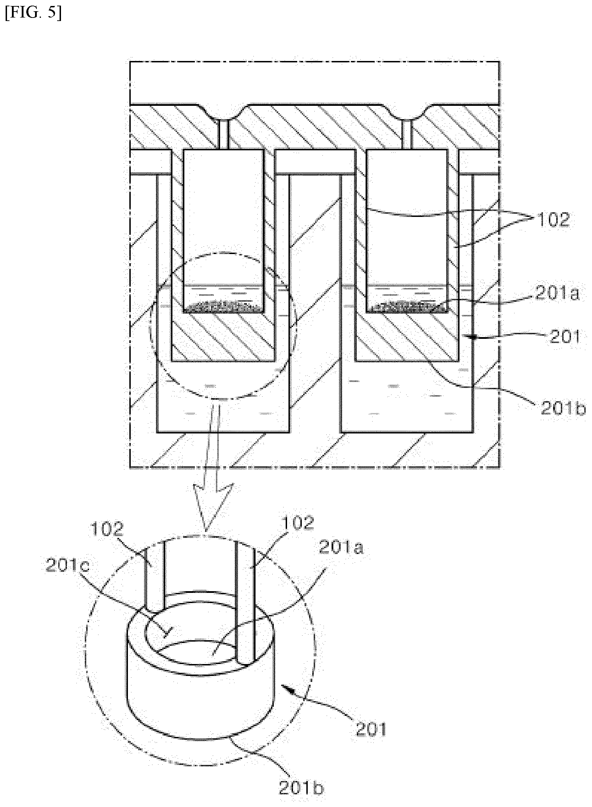

Meanwhile, FIG. 5 is a cross-sectional view illustrating a pillar structure for a biochip according to another exemplary embodiment of the present invention.

In the exemplary embodiment illustrated in FIG. 5, a seating surface 201a of a pillar part 201 is formed between the holder parts 102 and a light irradiation surface 201b is provided at a position symmetrical to the seating surface 201a, unlike the exemplary embodiment illustrated in FIGS. 3 and 4.

In the present exemplary embodiment configured as described above, since the cell is placed on the seating surface 201a formed between the holder parts 102, there is an advantage in that the cell may be stably positioned and fixed on the pillar part 201.

In addition, in the present exemplary embodiment, as well illustrated in an enlarged part in FIG. 5, the cell is positioned on the seating surface 201a by means of a stepped groove portion 201c formed on the pillar part 201, and as a result, there is an advantage in that the cell may be stably cultured without being withdrawn from the pillar part 201.

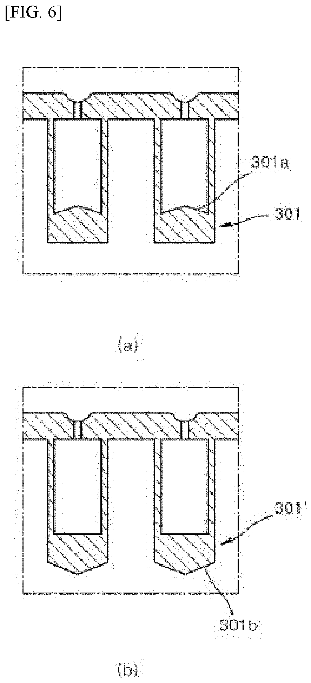

FIG. 6 is an enlarged cross-sectional view illustrating a configuration of a part of a pillar structure for a biochip according to still another exemplary embodiment of the present invention.

A pillar part 301 (301') employed in the present exemplary embodiment includes an inclined surface 301a (301b) formed on a light irradiation surface provided at a position symmetrical to a seating surface on which a cell is placed. The inclined surface 301a (301b) is provided to allow foreign substances to flow to the culture solution in order to prevent the foreign substances, such as some cells separated from the cells fixed to the seating surface, from remaining. The inclined surface is formed to be inclined so that a thickness thereof gradually decreases from a central portion of the light irradiation surface to a portion connected to the holder part.

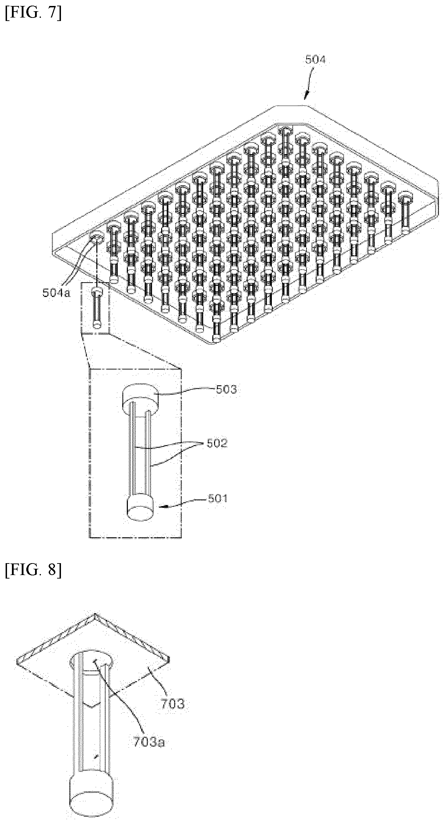

FIG. 7 is a bottom perspective view illustrating a pillar structure for a biochip according to yet another exemplary embodiment of the present invention.

The pillar structure for a biochip according to the present exemplary embodiment is manufactured separately from the substrate part 504 and then coupled to the substrate part 504, unlike the pillar structures according to the above-mentioned exemplary embodiments.

That is, the pillar structure for a biochip according to the present exemplary embodiment includes pillar parts 501, holder parts 502, and connection parts 503 which are manufactured separately from the substrate part 504 and formed integrally with one another. The pillar parts 501 is a portion on which the cell is placed. The pair of holder parts 502 is portions capable of receiving a relatively large amount of culture solution. The connection part 503 is a portion serving as a medium for coupling the holder parts 502 to the substrate part 504 according to the present exemplary embodiment, and the connection part 503 is detachably coupled to the substrate part 504.

That is, the connection part 503 is fixed between a pair of fitting ribs 504a formed on the substrate part 504 in an interference fit manner, such that the connection part 503 according to the present exemplary embodiment may be connected to or separated from the substrate part 504 without using a separate fastening or unfastening means.

FIG. 8 is a view for explaining a through hole formed in the substrate part according to yet another exemplary embodiment of the present invention.

A through hole 703a formed in the substrate part 703 according to the present exemplary embodiment is larger than a hole formed in the exemplary embodiment illustrated in FIG. 3, such that air may be smoothly discharged during product molding, the cell placed on the pillar parts may be irradiated with light, or the cell may be observed.

While various exemplary embodiments of the present invention have been described above, the present exemplary embodiments and the drawings attached to the present specification clearly show only a part of the technical spirit included in the present invention. It will be apparent that all the modified examples and the specific exemplary embodiments, which may be easily inferred by those skilled in the art within the scope of the technical spirit included in the specification and the drawings of the present invention, are included in the scope of the present invention.

## Claims

1. A pillar structure for a biochip, which is provided to form a biochip for analyzing a sample, which is a biological micromaterial such as a cell, together with a well plate configured to receive a culture solution, the pillar structure comprising:
a pillar part having a seating surface on which a cell for culture is placed, and a light irradiation surface configured to be irradiated with light for observing the cell placed on the seating surface; and
a plurality of holder parts extending in a state of being spaced apart from the pillar part and having an opening between the adjacent holder parts so that the culture solution is introduced between the holder parts.

2. The pillar structure of claim 1, wherein the light irradiation surface of the pillar part is formed between the holder parts, and the seating surface is provided at a position symmetrical to the light irradiation surface.

3. The pillar structure of claim 1, wherein the seating surface of the pillar part is formed between the holder parts, and the light irradiation surface is provided at a position symmetrical to the seating surface.

4. The pillar structure of claim 2 or 3, wherein the light irradiation surface is formed to be inclined so that a thickness thereof gradually decreases from a central portion to a portion connected to the holder part.

5. The pillar structure of claim 1, wherein the holder parts are formed integrally with a substrate part positioned opposite to the pillar part with the holder parts interposed therebetween, and the substrate part has a plate-shaped structure.

6. The pillar structure of claim 1, wherein the holder parts are manufactured separately from a substrate part having a plate-shaped structure and positioned opposite to the pillar part with the holder parts interposed therebetween, and
wherein the pillar structure further comprises a connection part serving as a medium for supporting the pillar part and the holder parts on the substrate part, the connection part being formed integrally with the pillar part and holder part and detachably coupled to the substrate part.

7. The pillar structure of claim 1, wherein a substrate part having a plate-shaped structure and positioned opposite to the pillar part with the holder parts interposed therebetween has a through hole formed at a position facing the pillar part.
